# EUROPEAN PATENT APPLICATION

(11) **EP 4 322 085 A1**
(43) Date of publication of application: **14.02.2024**
(21) Application number: 23190596.9
(22) Date of filing: 09.08.2023
(51) Int. Cl.: G06Q 10/0635

(54) **METHOD AND APPARATUS FOR SHARING DATA IN INTELLIGENT PHARMACOVIGILANCE PLATFORM**

(30) Priority: 11.08.2022 KR 20220100286
(71) Applicant: Selta Square Co., Ltd., Seoul 06168 (KR)
(72) Inventor: SHIN, Min Kyung, 18477 Gyeonggi-do (KR); YANG, Sol Ji, 06540 Seoul (KR)
(74) Representative: Peterreins Schley

(57) **Abstract**

For collaboration between servers in an intelligent pharmacovigilance (PV) platform, a method of operating a server for providing a pharmacovigilance (PV) platform and managing a first database (DB) of a first member company and a second DB of a second member company may comprise obtaining information on an agreement between the first member company and the second company for a product, establishing a connection for data sharing between the first DB and the second DB, setting a rule for data sharing and performing control to share data related to the product between the first DB and the second DB based on the rule.

## Description

### CROSS REFERENCE TO RELATED APPLICATION

The present application claims priority to Korean Patent Application No. 10-2022-0100286 filed on August 11, 2022, the entire contents of which is incorporated herein for all purposes by this reference.

### BACKGROUND OF THE INVENTION

### 1. Field of the Invention

The present invention relates to an intelligent pharmacovigilance (PV) platform, and more particularly, to a method and apparatus for sharing data in an intelligent pharmacovigilance platform.

### 2. Description of the Related Art

Pharmacovigilance (PV) refers to a task of monitoring clinical trial results and postmarketing safety information, such as the effects and side effects of new drugs. Pharmacovigilance also includes monitoring adverse drug reaction (ADR) that occur after marketing as well as during clinical trials and reporting them to relevant government departments.

Pharmaceutical companies that develop and market drugs need to perform pharmacovigilance procedures. As a full member of the International Conference on Harmonization (ICH), the pharmaceutical industry needs to follow the ICH guidelines in each field, including manufacturing and clinical trials. In particular, since pharmacovigilance regulations have been strengthened starting in Europe since 2012, pharmacovigilance is emerging as an important task in pharmaceutical companies that need to establish their own pharmacovigilance system.

Pharmacovigilance procedures require a variety of tasks, such as the collection of data from a variety of sources, the interpretation of unstructured data, and the application of regulations. In addition, in pharmacovigilance procedures, it is common for each member to have various authorities, and the content and scope of a given task vary depending on the authority. Therefore, the pharmacovigilance procedure has a very complex structure and requires a lot of manpower and cost to operate.

### SUMMARY OF THE INVENTION

The present invention is related to provide a method and apparatus for efficiently performing a pharmacovigilance (PV) procedure.

The present invention is related to provide a method and apparatus for performing a pharmacovigilance procedure based on an artificial intelligence algorithm.

The present invention is related to provide a method and apparatus for collaborating a pharmacovigilance procedure between different databases (DBs).

The present invention is related to provide a method and apparatus for exchanging data on one drug between different DBs.

The present invention is related to provide a method and apparatus for sharing data on one drug between different servers.

The present invention is related to provide a method and apparatus for enabling different DBs to perform a series of procedures for managing one drug.

The present invention is related to provide a method and apparatus for determining division of tasks for managing one drug between different DBs based on the contents of a license agreement.

The present invention is related to provide a method and apparatus for establishing a procedure for managing one drug by different servers based on the contents of a license agreement.

The technical problems solved by the present invention are not limited to the above technical problems and other technical problems which are not described herein will become apparent to those skilled in the art from the following description.

A method of operating a server for providing a pharmacovigilance (PV) platform and managing a first database (DB) of a first member company and a second DB of a second member company according to an embodiment of the present disclosure may comprise obtaining information on an agreement between the first member company and the second company for a product, establishing a connection for data sharing between the first DB and the second DB, setting a rule for data sharing, and performing control to share data related to the product between the first DB and the second DB based on the rule.

A server for providing a pharmacovigilance (PV) platform and managing a first database (DB) of a first member company and a second DB of a second member company according to an embodiment of the present invention may comprise a storage configured to store information necessary for operation of the server, a communication unit configured to transmit and receive data, and a processor connected to the storage and the communication unit. The processor may obtain information on an agreement between the first member company and the second company for a product, establish a connection for data sharing between the first DB and the second DB, set a rule for data sharing, and perform control to share data related to the product between the first DB and the second DB based on the rule.

The features briefly summarized above with respect to the present invention are only exemplary aspects of the detailed description of the present invention that follows, and do not limit the scope of the present invention.

### BRIEF DESCRIPTION OF THE DRAWINGS

The above and other objects, features and other advantages of the present invention will be more clearly understood from the following detailed description taken in conjunction with the accompanying drawings, in which:
FIG. 1 illustrates a structure of a system for providing a pharmacovigilance platform according to an embodiment of the present disclosure;
FIG. 2 illustrates the structure of a device according to an embodiment of the present invention;
FIG. 3 illustrates the structure of an artificial neural network applicable to a system according to an embodiment of the present invention;
FIG. 4 illustrates a functional configuration of a server according to an embodiment of the present invention;
FIG. 5 illustrates examples of tasks for processing adverse event data according to an embodiment of the present invention;
FIG. 6 illustrates an example of a procedure for sharing data in a pharmacovigilance platform according to an embodiment of the present invention;
FIG. 7 illustrates an example of a procedure for transmitting data in response to an event in a pharmacovigilance platform according to an embodiment of the present invention;
FIG. 8 illustrates an example of an interworking procedure between DBs for sharing data in a pharmacovigilance platform according to an embodiment of the present invention;
FIG. 9 illustrates an example of a procedure for transmitting data in response to an event in a pharmacovigilance platform according to an embodiment of the present invention; and
FIG. 10 illustrates an example of a procedure for processing adverse event data in a pharmacovigilance platform according to an embodiment of the present invention.

### DESCRIPTION OF THE PREFERRED EMBODIMENTS

Hereinafter, with reference to the accompanying drawings, embodiments of the present invention will be described in detail so that those skilled in the art can easily carry out the present invention. However, the present invention may be embodied in many different forms and is not limited to the embodiments described herein.

In describing the embodiments of the present invention, a detailed description of known functions and configurations will be omitted when it may obscure the subject matter of the present invention. In addition, in the drawings, portions which are not related to the description of the present invention will be omitted and similar portions are denoted by similar reference numerals.

The present invention proposes a technology for performing a pharmacovigilance (PV) procedure using an intelligent artificial intelligence platform. Specifically, the present invention relates to a technology for jointly processing a series of management procedures for one drug between member companies having different databases (DBs). That is, the present invention proposes various embodiments for supporting networking between DBs for exchanging safety information related to tasks to be performed according to a drug agreement.

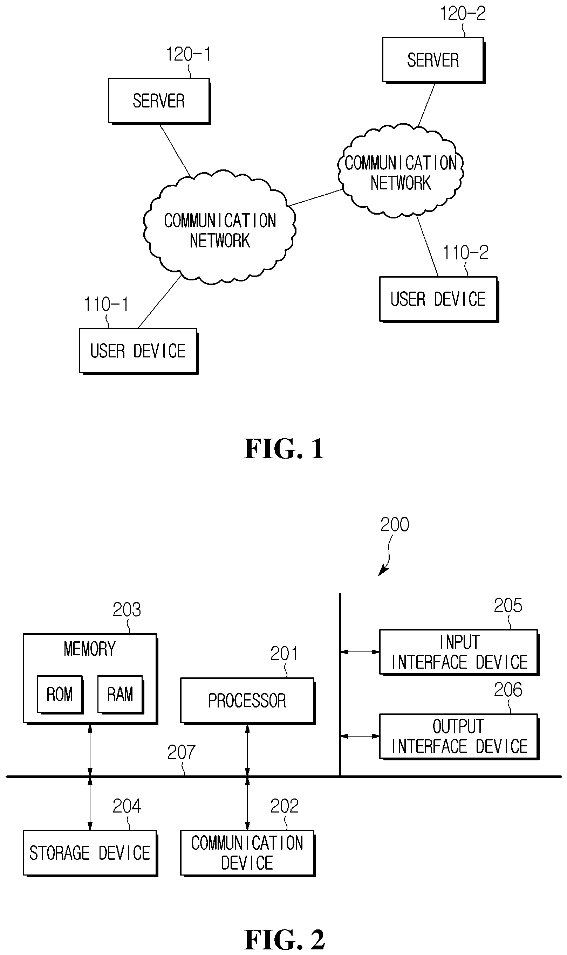

FIG. 1 illustrates a structure of a system for providing a pharmacovigilance platform according to an embodiment of the present disclosure.

Referring to FIG. 1, the system includes a first user device 110-1, a second user device 110-2, a first server 120-1, and a second server 120-2 connected to a communication network. Although FIG. 1 illustrates two user devices 110-1 and 110-2, three or more user devices may exist. Also, although FIG. 1 illustrates two servers 120-1 and 120-2, three or more servers may exist.

The first user device 110-1 and the second user device 110-2 are devices used by users who want to participate in a pharmacovigilance procedure using the platform according to an embodiment of the present invention. The first user device 110-1 and the second user device 110-2 may obtain input data (e.g., e-mail, document image, user input), transmit the input data to the first server 120-1 or the second server 120-2 through the communication network, and interact with the first server 120-1 or the second server 120-2. Each of the user devices 110-1 and 110-2 may include a communication unit for communication, a storage unit for storing data and programs, a display unit for displaying information, an input unit for user input, and a processor for control. For example, each of the user devices 110-1 and 110-2 may be a general-purpose device (e.g., smartphone, tablet, laptop computer, desktop computer) or a platform-specific access terminal in which an application or program for platform access is installed.

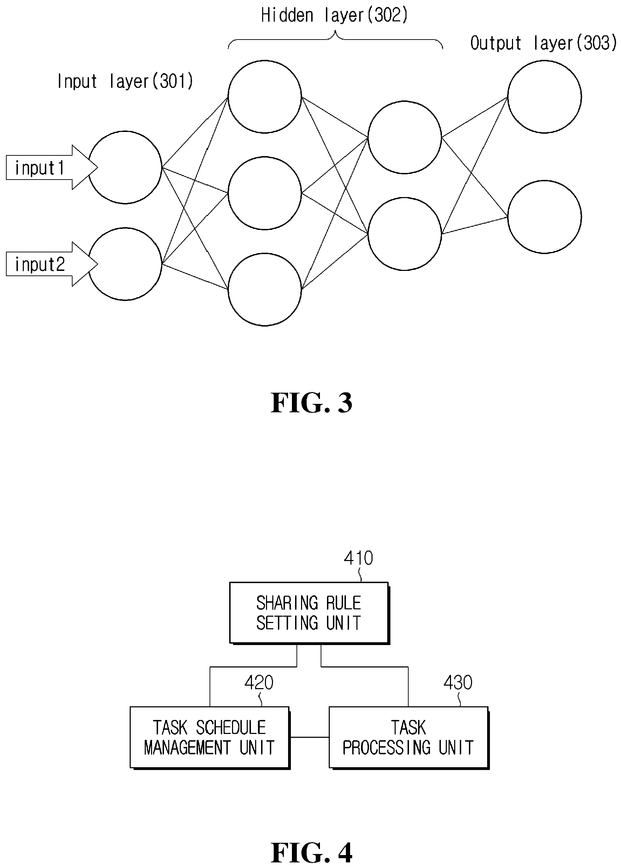

The first server 120-1 and the second server 120-2 provide a platform according to embodiments of the present invention. The first server 120-1 and the second server 120-2 provide various functions for the pharmacovigilance platform and may operate an artificial intelligence model. An example of an artificial neural network applicable to the present invention will be described with reference to FIG. 3 below. In addition, the first server 120-1 and the second server 120-2 may perform learning for an artificial intelligence model using learning data. According to various embodiments of the present invention, the first server 120-1 and the second server 120-2 may store a plurality of artificial intelligence models for various tasks included in the pharmacovigilance procedure, and selectively use at least one of artificial intelligence models as needed. In addition, the first server 120-1 and the second server 120-2 may store a plurality of visualization tools for presenting analysis results to users, and selectively use at least one of the visualization tools as needed. In addition, the first server 120-1 and the second server 120-2 may store profile information including authorities for a plurality of users and process input data based on authorities for a requested task. The first server 120-1 and the second server 120-2 may be local servers existing in a local network or may be remote access servers (e.g., cloud servers) connected through an external network. The first server 120-1 and the second server 120-2 may include a communication unit for communication, a storage unit for storing data and programs, and a processor for control.

The first server 120-1 and the second server 120-2 may include DBs managing data of different member companies, respectively. The DBs included in the first server 120-1 and the second server 120-2 are managed under the control of a system provider, and operation of the first server 120-1 or the second server 120-2 described later may be understood as the operation of the system provider or the operation of the DB under the control of the system provider. For example, the first server 120-1 may include a first DB for managing data of a first member company, and the second server 120-2 may include a second DB for managing data of a second member company. Alternatively, the first server 120-1 may include a first DB managing data of a first member company and a second DB managing data of a second member company, and the second server 120-2 may include a third DB managing data of a third member company. That is, depending on the implementation, DBs of different member companies may be included in one server. However, even if DBs are included in one server, they may be treated as logically separated.

Here, the member company refers to an object capable of independently performing a pharmacovigilance procedure, and may correspond to, for example, one pharmaceutical company. Similarly, branch offices of a company in different countries may correspond to the first server 120-1 and the second server 120-2. Member companies in the partnership are separate network modules and have different DBs. Therefore, setting through authentication is required when sharing data. On the other hand, branch offices are subgroup modules, which are subcategories of member companies. Therefore, accessible data may be limited by a method of imposing function restrictions on the headquarters DB rather than data sharing between DBs, and separate authentication is not required even if data is shared.

User types provided by the platform according to embodiments of the present invention may be classified as follows. First, as a general user, it is a user who can perform data input and processing for the DB to which they belong. Second, as an administrator user, it is a user who can perform data input and processing for the DB to which they belong and has authority such as some settings including user registration. Third, as a system administrator, it is a user belonging to the developer of the platform, who is involved in maintenance/repair operations and has the authority to change all DBs. However, the system administrator does not have the authority to manage input data in the individual DB, and can handle the data only at the request of the user belonging to the DB.

According to various embodiments, data stored in a first DB may be shared with a second DB. For example, data may include information related to the safety of a drug. In the case of data sharing, conditions, rules, and restrictions for sharing may be set according to a predefined protocol between DBs. That is, data may be shared in a set situation, only for a specific drug according to a set rule or the like.

FIG. 2 illustrates the structure of a device according to an embodiment of the present invention. The device 200 is a functional unit that uses a common storage space for data necessary for program execution. The device 200 may include at least one computer and software associated therewith. The device 200 may be understood as a structure of the user device 110-1 or 110-2 or the server 120-1 or 120-2 of FIG. 1.

Referring to FIG. 2, the device 200 may include at least one of a processor 210, a communication device 220, a memory 230, a storage device 240, an input interface device 250, or an output interface device260, all of which perform communication through a bus 270.

The processor 210 is hardware having a function of processing and/or processing various types of information within the device 200. The processor 210 may be a semiconductor device that executes commands stored in a central processing unit (CPU), the memory 230 and/or the storage device 240.

The communication device 220 is a data transmission device for exchanging data with other devices or systems in data communication. The communication device 220 may include a data input/output device or a communication control device. For example, the communication device 220 enables communication of voice, video, and text data between the data system and other devices.

The memory 230 is a storage device capable of storing information. The information includes programs or software necessary for the operation of the device 200, data generated during operation, and the like. The memory 230 may include a read only memory (ROM) and a random access memory (RAM). Here, the RAM may load data, process what is necessary, and store changes back. The ROM is a read-only storage device, and data stored in the ROM may be stored permanently or semi-permanently.

The storage device 240 may store various types of information processed in the device 200. The storage device 240 may include various types of volatile or non-volatile storage media.

The input interface device 250 may detect commands from the user and allow the user to operate the system. In addition, the output interface device 260 may display the result of the user's use of the system. The input interface device 250 and the output interface device 260 may be user interfaces (UIs).

The steps of a method or algorithm described in connection with the embodiments described herein may be directly embodied in a hardware or software module executed by the processor 210, or a combination of the two. A software module may be reside in a storage medium (i.e., the memory 230 and/or the storage device 240) such as a RAM memory, a flash memory, a ROM memory, an erasable programmable read only memory (EPROM), an electrically erasable programmable read only memory (EEPROM), a register, a hard disk, a detachable disc or a CD-ROM.

An exemplary storage medium is coupled to the processor 210, and the processor 210 may read information from the storage medium, and write information to the storage medium. Alternatively, the storage medium may be integral with the processor 210. The processor 210 and the storage medium may reside within an application specific integrated circuit (ASIC). The ASIC may reside in a user terminal. Alternatively, the processor and the storage medium may reside in a user terminal as separate components.

FIG. 3 illustrates the structure of an artificial neural network applicable to a system according to an embodiment of the present invention. The artificial neural network as shown in FIG. 3 may be understood as a structure of an artificial intelligence model stored in the server 120-1 or 120-2. Referring to FIG. 3, the artificial neural network includes an input layer 301, at least one hidden layer 302, and an output layer 303. Each of the layers 301, 302, and 303 is composed of a plurality of nodes, and each node is connected to output of at least one node belonging to a previous layer. Each node calculates an inner product of an output value of each of the nodes in the previous layer and a connection weight corresponding thereto, and then sends an output value multiplied with a non-linear activation function to at least one neuron in a next layer.

The artificial neural network shown in FIG. 3 may be formed by learning (e.g., machine learning, deep learning, etc.). In addition, artificial neural network models used in various embodiments of the present invention may include at least one of a fully convolutional neural network, a convolutional neural network, a recurrent neural network, a restricted Boltzmann machine (RBM) or a deep belief neural network (DBN), but is not limited thereto. Alternatively, machine learning methods other than deep learning may also be included. Alternatively, a hybrid model which is a combination of deep learning and machine learning may also be included. The machine learning-based model may include a Support Vector Machine (SVM), AdaBoost, and the like, but is not limited thereto.

FIG. 4 illustrates a functional configuration of a server according to an embodiment of the present invention. The functional configuration of FIG. 4 may be understood as a structure of the server 120-1 or 120-2.

Referring to FIG. 4, the server may include a sharing rule setting unit 410, a task schedule management unit 420, and a task processing unit 430.

The sharing rule setting unit 410 sets a rule for data sharing. The rule is defined to specify a condition and a data item corresponding to the condition. The rules may be set based on agreement information. The agreement information may be input by a user through a platform or webpage or extracted from an electronic document uploaded through the platform or webpage. Additionally, the sharing rule setting unit 410 may set a scope of shared data based on the agreement information. Here, setting the scope of shared data may be understood as being included in setting the rule.

The task schedule management unit 420 schedules various tasks included in the pharmacovigilance procedure and monitors whether the tasks are performed according to the scheduling. If necessary, the task schedule management unit 420 may request confirmation of the progress of the task from a user device or the counterpart server.

The task processing unit 430 performs at least some of tasks included in the pharmacovigilance procedure and tasks related thereto. Here, performing the task may include performing an operation necessary for the task using a prepared algorithm, providing a necessary interface to a user having authority, requesting the progress of the task, and the like.

The scope of sales of drugs may be expanded through agreement. In particular, when selling drugs to other countries, companies may enter into a license agreement with a local pharmaceutical company. In this case, an original company supplying the drug and a local partner company receiving the drug become parties to the agreement. The license agreement becomes a license-out agreement from the standpoint of the original company and a license-in agreement from the standpoint of the local partner company. When selling drugs, there is an obligation to report safety information generated domestically and internationally in accordance with relevant national regulations, and accordingly, the exchange of safety information collected about drugs is necessary. To this end, a safety data exchange agreement, which is a sub-agreement of a license agreement, may be made. The safety data exchange agreement (SDEA) is also called a pharmacovigilance agreement (PVA). The SDEA or the PVA agreements may be made not only for license agreements, but also for joint sales, joint development, and joint research.

Pharmacovigilance procedures may be carried out for drugs that are the subject of a license agreement. In this case, it may be necessary to exchange information about the drug between member companies, which are parties to the agreement. This exchange of information may be understood as data exchange between servers or data exchange between DBs. In general, data exchange between DBs may be performed through a gateway, but this requires high cost and may cause loopholes in data security. Accordingly, the present invention describes various embodiments capable of exchanging data rapidly and accurately while maintaining security under strict management.

A company that manufactures and sells drugs under a license agreement may have different obligations for drugs depending on the type and contents of the agreement. For example, a market authorization holder (MAH) has permission in a corresponding country and has an obligation to report safety information of a corresponding drug to a regulatory authority. Here, the MAH may be referred to as `a person who has obtained marketing approval or filed marketing notification pursuant', 'a person holding marketing approval', 'an item permission holder', 'a person who has obtained item permission of drugs', and the like. A distributor is an entity that only sells drugs within the corresponding country and does not have an obligation to report to the regulatory authority. Looking at the obligations of the MAH and distributor in terms of data exchange, the MAH and distributor require different operations on data. Specifically, since the MAH has obligations to report overseas cases as well, it is necessary to provide and collect data. On the other hand, since the distributor does not have direct reporting obligations, it is necessary to provide data.

An SDEA may be made as a sub-agreement of the license agreement. The SDEA is also called a PVA. The SDEA may include various clauses, for example, data scope for exchanging and managing safety information, including adverse events collected for agreed drugs, and report timing for cases, role and responsibility (RNR) of each party, etc. may be specified. For example, the report timing may be set within a certain time (e.g., 24 hours) from the date of receipt or awareness of the safety information. Here, the safety information includes all information about the corresponding drug collected through pharmacovigilance. The safety information mainly includes reports of side effects and adverse events, but may additionally or alternatively include pregnancy, off-label use, misuse, abuse, medication errors, occupational exposure, drug addiction, drug-borne infections, quality problems, ineffectiveness, important licensing issues and the like according to the agreement. In addition, the SDEA may specify the performance of reconciliation task, the creation and exchange of periodic safety aggregated data documents, etc. Here, the reconciliation task is a procedure of comparing and checking the history of data exchange (e.g., data transmission, data reception) with the other party's record, and may be performed, for example, monthly, quarterly, semiannually, or annually.

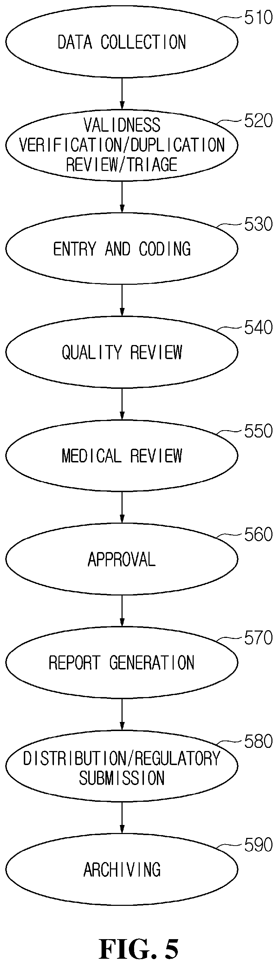

According to the contents of the agreement as described above, adverse event data among safety information may be processed through a series of tasks as shown in FIG. 5 below. FIG. 5 illustrates examples of tasks for processing adverse event data according to an embodiment of the present invention. Referring to FIG. 5, adverse event data may be processed through data collection task 510, validness verification/duplication review/triage task 520, entry and coding task 530, quality review task 540, medical review task 550, approval task 560, report generation task 570, distribution/regulatory submission task 580, and archiving task 590. At least one of the listed tasks may be repeated as needed.

In the data collection task 510, adverse event data is obtained. The adverse event data includes information about the occurrence of reactions other than the originally targeted reactions or effects, such as side effects for the drug. The adverse event data may be collected through various channels. The data collection task 510 may be understood to include delivery of an acknowledgment (ACK) or receipt for the collected information. The data collection task 510 may be referred to as 'intake and receipt'.

In the validness verification/duplication review/triage task 520, it is checked whether or not the collected adverse event data includes essentially required information items (hereinafter referred to as 'essential items'), and checking duplication with and triage of already collected adverse event data may be performed based on the essential items. For example, the essential items may include patient information, reporter information, drug information, adverse reaction degree, and the like. That is, if it includes 4 items: information on one identifiable patient, suspected drug information, adverse reactions/events (usually referred to as adverse reactions during clinical trials and adverse events after marketing), and reporter information, the data may be considered as a valid case. In addition, according to an algorithm based on the comparison of essential items and important additional information such as date information, it is determined whether the adverse event overlaps previously collected and processed adverse events, and if not, it is triaged for subsequent processing. Triage includes triage according to seriousness, causality, and predictability of reported adverse event. Depending on the triage result, the deadline for delivery to the regulatory authority or partner company may vary, and the deadline may be controlled so that violations do not occur by applying relatively conservative triage criteria first.

In the entry and coding task 530, the adverse event data is input and coded according to the format provided by the pharmacovigilance platform. At this time, the user inputs various information including essential items included in the adverse event data through an interface provided by the pharmacovigilance platform. At least some of the input data is processed through coding. For example, various expressions referring to the same object, symptom or phenomenon may be converted into a single designated expression. According to an embodiment, conversion of expression, i.e., coding, may be performed based on an internationally used term list or drug list.

In the quality review task 540, the quality of the input information is reviewed. Specifically, data completion through a query issue and information input in a previous step are reconfirmed, and appropriateness of coding may be evaluated. In addition, predictive evaluation may be performed by comparing drug approvals and reported adverse events. However, evaluation of quality issues and predictability is not limited to quality review task, and may be performed according to the workflow set by member companies.

In the medical review task 550, a review by users consisting of pharmaceutical experts and the like may be performed. Users capable of performing the medical review task 550 may be identified through user qualification in the DB. In the medical review task 550, a medical evaluation and causality evaluation of the safety information may be performed. In the causality evaluation task, the causal relationship between the use of the target drug and the occurrence of an adverse reaction is evaluated. In the causality evaluation task, at least one user's judgment may be involved, and data may be provided to the user device for review by the user. Causality evaluation may be performed as part of other tasks, depending on the workflow established by the member company.

In the approval task 560, a final inspection of the results of the previous tasks 520, 530, 540, and 550 is performed. The final inspection may be performed by an algorithm or by a user with authority (e.g., CL (case lock)). To this end, summary information of individual adverse events and data on the results of causality, predictivity, and materiality evaluation in the previous step may be provided to the user device in the workflow.

In the report generation task 570, a report on the adverse event is generated for external submission. The report may be prepared to include information on evaluation and information checked in previous tasks. The report may be generated by an algorithm provided by the pharmacovigilance platform, and, for example, an artificial intelligence algorithm may be used.

In distribution/regulatory submission task 580, the completed report is transmitted to other member companies or submitted to regulatory authorities. Submission of the report may be performed through a communication network or offline after output. However, this task may be omitted in some cases.

The archiving task 590 is a step of locking report processing. Accordingly, data may be managed in an unmodifiable state, and reports and related data are stored in an archive.

In the procedure including the nine steps described with reference to FIG. 5, as an example, the order of some tasks may be changed or a specific task may be skipped according to pharmaceutical companies, drugs, and the seriousness of adverse events. Also, in the DB, it is possible to modify the workflow according to each customer's internal policy.

According to the definition of the procedure, the tasks up to the report generation task 570 among the series of tasks in FIG. 5 may be performed by an object functioning as a distributor (hereinafter referred to as a 'first object') and the regulatory submission task 580 may be performed by an obj ect functioning as a MAH (hereinafter referred to as 'second obj ect'). In this case, the second object cannot even recognize the occurrence of the adverse event until the report is generated. However, according to various embodiments of the present invention, it is possible to rapidly exchange data between two objects through a pharmacovigilance platform. Accordingly, according to setting of appropriate rules, even before the report generation task 570, the second object may recognize the occurrence of the adverse event and participate in the management of the task schedule. As a specific example, when the first object starts the validness verification/duplication review/triage task 520, the start may be notified to the second object. In addition, related data may be shared with the second object in order for the second object to perform at least a part of a specific task according to a rule set in advance. Specifically, the second object may additionally perform causality evaluation by additionally allocating the quality review task 540 or the medical review task 550 to the second object. In addition, when the report generation task 570 is completed, a rule may be set so that report data is immediately transmitted to the second obj ect, and accordingly, the second object may recognize the completion of the report without delay.

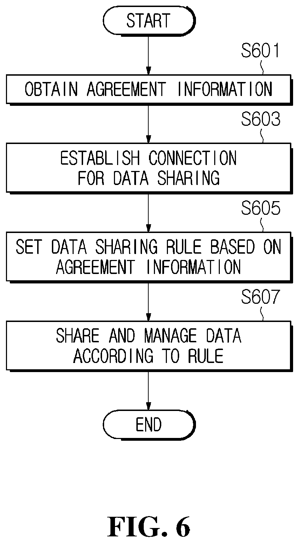

FIG. 6 illustrates an example of a procedure for sharing data in a pharmacovigilance platform according to an embodiment of the present invention. FIG. 6 illustrates an operating method of a subject requesting conclusion of an agreement. In the description of FIG. 6, the operating subject may be a server (e.g., the first server 120-1 of FIG. 1) that manages DBs. Alternatively, the operations illustrated in FIG. 6 may be understood as operations of a DB.

Referring to FIG. 6, in step S601, the server obtains agreement information. The agreement information may be generated as an agreement is concluded offline or through a separate platform for agreement conclusion. When an agreement is concluded offline, agreement information may be obtained by notifying each other of agreement information between managers of DBs belonging to agreement subjects. When a separate platform is used, the agreement may be made in the form of an electronic agreement, and agreement information may be shared with DBs. That is, agreement information may be input and stored through the server, or may be obtained through interworking with a separate platform operated in another server. To this end, the server or the separate platform provides an interface for inputting agreement information to the user device, and the user device may transmit information input by the user to the server through the interface. For example, agreement information may include information on the target drug, the triage of the agreement (e.g., whether or not it is permitted), the scope of safety information to be reported, the reporting deadline, reporting form, information on person in charge, and pharmacovigilance procedure information. At this time, the server may store data on the contents of the agreement in the DB.

In step S603, the server establishes a connection for data sharing. The server may establish at least one link or channel for data sharing between DBs. According to an embodiment, when agreement information is obtained and member companies confirm the agreement information, the agreement information is transmitted to a system provider, and a connection may be established under the control of the system provider. That is, a connection may be established under the control of a system provider, and a specific connection procedure may vary depending on a cloud where a server is located. For example, in the case where connection between DBs is performed through an application programming interface (API), when the system provider receives the result of agreement information from each member company, the connection may be controlled by issuing an API authentication key to each DB. However, the present invention is not limited to this, and various methods such as a connection through a link, a gateway, a relay server managed by a system provider, or a connection through a separate layer accessible only by a system administrator in a multi-tier architecture may be applied.

In step S605, the server sets a data sharing rule based on the agreement information. The agreement contains the contents of the pharmacovigilance procedure and may stipulate the distribution of tasks necessary for pharmacovigilance for the drug. Accordingly, according to an embodiment, the server determines the rule including a condition, timing, and a period for data sharing based on the agreement information, and stores information about the rule. According to another embodiment, the server may provide member companies or system providers with an interface for setting the sharing rule, determine and store the sharing rule according to data input through the interface. Additionally, in addition to data sharing, rules for other operations related to data (e.g., notification of occurrence, restriction on sharing, change of authority, etc.) may be further determined.

In step S607, the server shares and manages data according to the rule. When a connection is established, data sharing between the server and the counterpart server becomes possible. When safety-related data (e.g., adverse event data) for a specific drug is generated, a series of procedures for pharmacovigilance are performed according to the previously set sharing rule, and, in this case, the server may receive or transmit data from or to the counterpart server, for processing of each task. In addition, the server may record a history of transmission and reception of data and perform necessary subsequent operations.

The sharing rule is set according to the procedure described with reference to FIG. 6, and data may be shared according to the set sharing rule. In this case, according to an embodiment, sharing conditions and timing may be defined as shown in [Table 1] below.

**[Table 1]**

| Type | Contents |
|---|---|
| Sharing conditions | - list of product names of drugs suspected for adverse event, ingredient names, and other product names sold worldwide |
| | - Sources from which adverse events were collected, whether they were reported voluntarily, whether they were collected from investigations and studies, including clinical trials, and whether they were collected through literature review, etc. |
| | - Whether the adverse event is serious (e.g., death, fatal case, hospitalization, teratogenicity, disability, other medically important cases, etc.), whether it is causal with drugs, whether it is predictive |
| | - adverse event of special interest: whether it is an adverse event that does not meet the criteria for a serious adverse event but requires special continuous monitoring |
| | - Whether it is a valid report (e.g., whether it contains 4 essential information) |
| | - Whether it is a special situation (pregnancy, off-label use, misuse, misuse, medication error, occupational exposure, drug addiction, drug-borne infection, quality problems, ineffectiveness, etc.) |
| Timing and period | - From the date of recognition to when, respectively, on the calendar day basis and business day basis |
| | - Whether the date of recognition is included in a specific investigation period and a report is required |
| | - In the case of a research study, whether it is reported on the date of submission of the report |

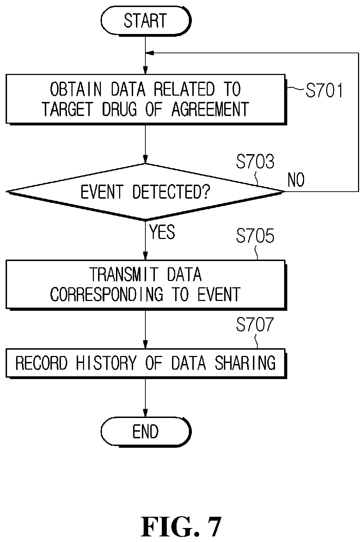

FIG. 7 illustrates an example of a procedure for transmitting data in response to an event in a pharmacovigilance platform according to an embodiment of the present invention. FIG. 7 illustrates an operation method of a subj ect providing data according to the contents of an agreement. In the description of FIG. 7, the operating subject may be a server that manages DBs (e.g., the first server 120-1 or the second server 120-2 of FIG. 1). Alternatively, the operations illustrated in FIG. 7 may be understood as operations of a DB.

Referring to FIG. 7, in step S701, the server obtains data related to the target drug of the agreement. For example, the data related to the target drug may include at least one of raw data on cases related to the drug received from the outside, information on the progress of a drug-related task, information on control of the progress of a drug-related task, or the result of the task related to the drug.

In step S703, the server checks whether an event is detected. The server determines whether the contents of the obtained data or obtaining of the data corresponds to any one of the events defined in the sharing rule. That is, the server checks whether the operation of step S701, the data obtained in step S701, the situation when obtaining the data, and the like meet the conditions of the event.

When an event is detected, in step S705, the server transmits data corresponding to the event. That is, the server transmits data as an operation corresponding to an event defined in a sharing rule. At this time, the server transmits data including information specified in the sharing rule. For example, the server may transmit the obtained data or data derived from the obtained data. The data derived from the obtained data may be one of a notification notifying the obtaining of data and data obtained by processing the obtained data.

In step S707, the server records a history of data sharing. The server records the information contained in the data, when the data was transmitted, and the event that caused the transmission of the data. The record of data sharing may be used for later pharmacovigilance-related tasks (e.g., reconciliation). In addition, the record of data sharing may be used later to evaluate whether there has been data sharing violating the contents of the agreement.

According to the procedure described with reference to FIG. 7, data may be shared according to detection of various events. For example, when safety information collected from a particular drug meets a sharing rule, that data and data added in processing may be shared. Also, periodically accumulated data sharing records may be shared as additional data. As another example, when safety information suitable for the sharing rule is collected, when data is transmitted to another member company, when a reconciliation-related request and transmission operation is performed, when a person in charge is changed during data transmission and reception in the workflow, when the report is distributed, when the report is delayed, when the information (name, ingredient, dose, etc.) on the target drug of the agreement and the rule related thereto are changed, or when the agreement status is changed, notification may occur.

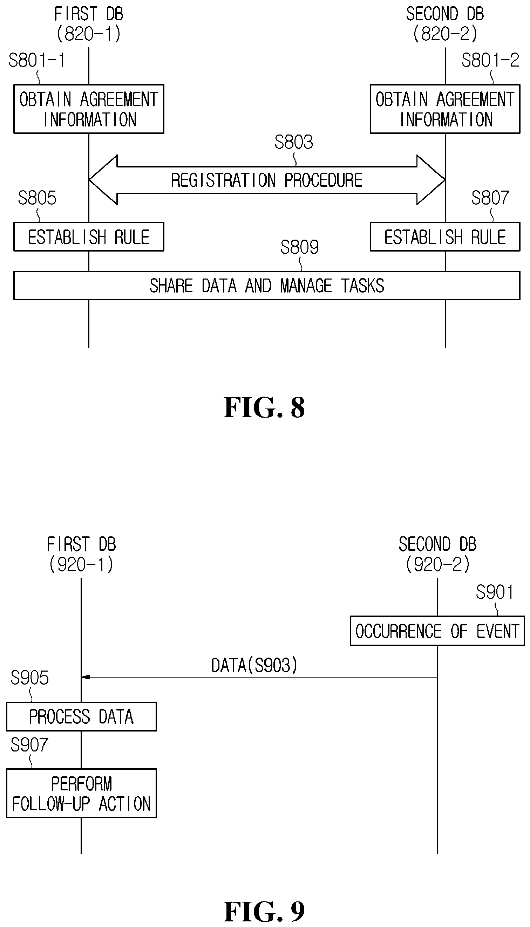

FIG. 8 illustrates an example of an interworking procedure between DBs for sharing data in a pharmacovigilance platform according to an embodiment of the present invention. FIG. 8 illustrates an interworking procedure between a first DB 820-1 and a second DB 820-2. The first DB 820-1 and the second DB 820-2 are DBs of different member companies, and may be two physically separated devices or logically separated objects within one hardware device.

Referring to FIG. 8, in steps S801-1 and S801-2, the first DB 820-1 obtains agreement information. According to various embodiments, the agreement information may be received from a user device accessing the first DB 820-1 or may be received from a platform located in a separate server for performing the agreement.

In step S803, the first DB 820-1 and the second DB 820-2 perform a registration procedure. Through the registration procedure, a connection for data exchange between the first DB 820-1 and the second DB 820-2 is established. For example, each of the first DB 820-1 and the second DB 820-2 may generate information necessary for mutual data exchange such as establishing a connection through a gateway for connection with the counterpart DB, and setting the allowable range of data viewing/request of the counterpart. In addition, the first DB 820-1 and the second DB 820-2 may negotiate information for security of shared data (e.g., information related to encryption/decryption (e.g., encryption key), etc.). These connection establishment operations may be performed under the control of a system provider.

In step S805, the first DB 820-1 establishes a rule. The first DB 820-1 determines conditions for data sharing with the second DB 820-2 and a rule specifying data to be shared according to the conditions. For example, the rule may be determined based on the agreement information obtained in step S801. The rule may further define follow-up actions for data sharing. As another example, the rule may be input from an authorized user or system provider.

In step S807, the second DB 820-2 establishes a rule. The second DB 820-2 determines conditions for data sharing with the first DB 820-1 and a rule specifying data to be shared according to the conditions. For example, the rule may be determined based on the agreement information obtained in step S801. The rule may further define follow-up actions for data sharing. As another example, the rule may be input from an authorized user or system provider.

In step S809, the first DB 820-1 and the second DB 820-2 share data and manage tasks. According to various embodiments, the first DB 820-1 and the second DB 820-2 monitor an event occurring while processing data (e.g., adverse event data) in the platform, and share data according to the detected event and the established rule. Also, the first DB 820-1 and the second DB 820-2 may manage tasks related to pharmacovigilance based on shared data. For example, the first DB 820-1 and the second DB 820-2 may monitor whether or not data is missing by performing a reconciliation procedure at regular time intervals according to a set period.

FIG. 9 illustrates an example of a procedure for transmitting data in response to an event in a pharmacovigilance platform according to an embodiment of the present invention. FIG. 9 illustrates signal exchange between a first DB 920-1 and a second DB 920-2. The first DB 920-1 and the second DB 920-2 have DBs of different member companies, and may be two physically separated devices or logically separated objects within one hardware device.

Referring to FIG. 9, in step S901, the second DB 920-2 recognizes occurrence of an event. The event is one of various events that may occur during pharmacovigilance, and may be related to at least one of various factors such as generation of data, processing of a task, user's command, and lapse of a period. The event is related to a condition causing data sharing, and the second DB 920-2 may detect the event based on various confirmed factors.

In step S903, the second DB 920-2 transmits data corresponding to the event to the first DB 920-1. The data corresponding to the event may be determined based on a rule for data sharing. That is, the second DB 920-2 may check a condition including the detected event among conditions included in the established rule, and check at least one data item requesting transmission in response to the condition. Then, the second DB 920-2 may read at least the checked data item and transmit a message for data sharing, including the read at least one data item, to the first DB 920-1. According to various embodiments, the message may include information indicating contents of the event, data generated in relation to the event, and the like. At this time, the message or data included in the message may be encrypted for security.

In step S905, the first DB 920-1 processes data. That is, the first DB 920-1 receives a message for data sharing and checks data included in the message. Also, the first DB 920-1 may store the data in its own DB and transmit the data to the user device of the related user. If necessary, the shared data may be processed after being processed through a process such as coding.

In step S907, the first DB 920-1 performs a follow-up action. The first DB 920-1 may perform a follow-up action related to the processed data. The follow-up action may be identified based on the rule for data sharing. That is, the follow-up action may also be understood as an action corresponding to a kind of event. For example, the first DB 920-1 may perform a task related to processed data among tasks related to pharmacovigilance. To this end, the first DB 920-1 may transmit necessary data to the user device of the user designated for the task.

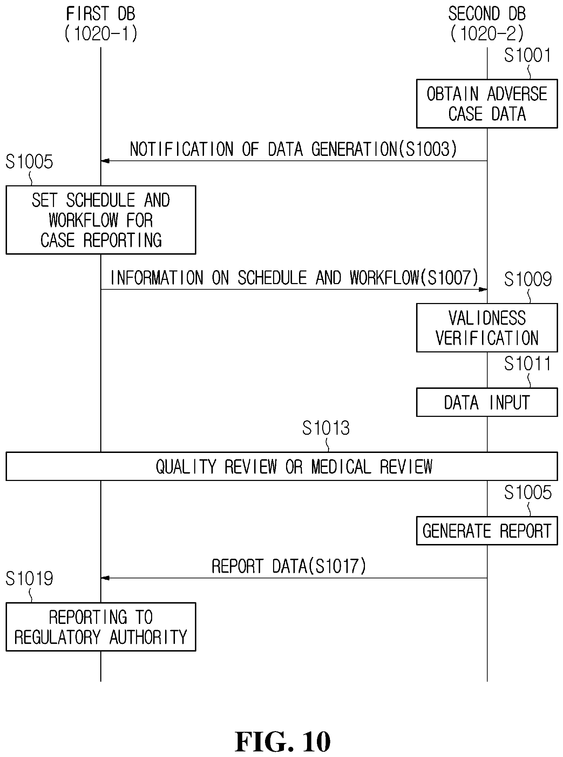

FIG. 10 illustrates an example of a procedure for processing adverse event data in a pharmacovigilance platform according to an embodiment of the present invention. FIG. 10 illustrates signal exchange between a first DB 1020-1 and a second DB 1020-2. The first DB 1020-1 and the second DB 1020-2 have DBs of different member companies, and may be two physically separated devices or logically separated objects within one hardware device.

Referring to FIG. 10, in step S 1001, the second DB 1020-2 obtains adverse event data. The adverse event data may be extracted from various types of files. For example, the second DB 1020-2 may receive an electronic document from a user device and extract information about the adverse event by analyzing the received electronic document.

In step S 1003, the second DB 1020-2 transmits a notification of data generation. In other words, the second DB 1020-2 receives a notification message notifying the first DB 1020-1 of the occurrence of an event of obtaining adverse event data. Through this, the first DB 1020-1 may confirm that an adverse event to be processed has occurred. According to various embodiments, the notification may include summary information of the adverse event.

In step S1005, the first DB 1020-1 sets a schedule and workflow for case reporting. Processing of an adverse event may be completed as a report to a regulatory authority. Accordingly, the first DB 1020-1 may check the tasks constituting the pharmacovigilance procedure for the adverse event and allocate each of the tasks to users having necessary authority. Also, the server may determine deadlines for each task. The deadlines may be used to transmit messages to confirm progress of the task in case of non-compliance. By setting the workflow, each of the first DB 1020-1 and the second DB 1020-2 may perform only designated tasks. The workflow may be set for each server or each user, and may include information specifying RNR.

In step S1007, the first DB 1020-1 transmits information on the set schedule and workflow to the second DB 1020-2. In other words, the first DB 1020-1 shares the schedule and workflow determined in step S1005 with the second DB 1020-2. Accordingly, the first DB 1020-1 and the second DB 1020-2 may have a common task scheduling for one adverse event. Accordingly, necessary tasks may be sequentially performed by collaboration of the first DB 1020-1 and the second DB 1020-2.

In step S1009, the second DB 1020-2 performs a validness verification task. That is, the second DB 1020-2 determines whether to proceed with the tasks for the adverse event confirmed in step S1001. Specifically, the second DB 1020-2 may check whether the adverse event data includes essential items and determine whether or not it overlaps the previously processed adverse event data. To this end, the second DB 1020-2 may use at least one artificial intelligence model.

In step S1011, the second DB 1020-2 performs a data input task. The second DB 1020-2 inputs adverse event data according to a predefined format. Since data is shared according to various embodiments, the adverse event data may be coded in a format defined in the platform and may be input. To this end, the second DB 1020-2 may use at least one artificial intelligence model.

In step S1013, the first DB 1020-1 and the second DB 1020-2 perform quality review or medical review. Each task may be performed by division of labor between users belonging to the first DB 1020-1 and users belonging to the second DB 1020-2. In this case, some of the sub-tasks included in the quality and medical review may be performed by the first DB 1020-1 and the other sub-tasks may be performed by the second DB 1020-2. Accordingly, results of sub-tasks may be transmitted between the first DB 1020-1 and the second DB 1020-2. For example, when the first task is completed by the second DB 1020-2, the completion of the first task is notified, and data notifying the completion of the first task and including the result of the first task is transmitted from the second DB 1020-2 to the DB 1020-1, and the second DB 1020-1 may perform a second task that follows based on the result of the first task. In this case, data including the result of the first task may be transmitted in response to identifying completion of the first task in the second server 1220-2 based on the set rule without user intervention.

In step S1015, the second DB 1020-2 performs a report generation task. A report is a document for reporting information about an adverse event to the outside (e.g. regulatory authority) and may be prepared to include information confirmed or analyzed through the steps performed above. To this end, the second DB 1020-2 may use at least one artificial intelligence model. According to another embodiment, the report may be generated by the first DB 1020-1.

In step S1017, the second DB 1020-2 transmits report data to the first DB 1020-1. That is, when the first DB 1020-1 has a report obligation, the generated report is transmitted to the first DB 1020-1. If the report is generated by the first DB 1020-1 according to another embodiment, this step may be omitted.

In step S1019, the first DB 1020-1 performs a regulatory authority reporting task. The reporting task may be done online or offline. When performed online, the first DB 1020-1 may generate a message including report data and transmit the generated message to an address on a network designated by a regulatory authority.

According to various embodiments described above, data may be shared based on an agreement for a specific drug, that is, a specific drug product. Procedures such as data sharing may be performed for each product. When a plurality of products are registered in a DB of one member company, a tree for products registered in the platform may be formed. The products may be grouped based on various attributes (e.g., active pharmaceutical ingredient(s) (API), which may be referred to as main ingredients, active ingredients, effective ingredients, raw material medicine, etc.) of the drug products and divided into high-level products and low-level products. This grouping and upper-level/lower-level division may be expressed in a tree structure, and if a user has data viewing rights for a certain product, viewing of lower-level products is also possible. Accordingly, accessible products may be limited by setting sites for each member company.

Through the various embodiments described above, sharing of data between servers of different member companies may be achieved. The function of data sharing provided by the pharmacovigilance platform according to an embodiment of the present invention is to organically support tasks (e.g., sharing of adverse event (e.g., side effects), data consistency task, and the like) with associated partner members for the purpose of maintaining the overall pharmacovigilance platform, as well as reporting to regulatory authorities, among the various tasks belonging to the pharmacovigilance procedure. That is, the platform according to an embodiment of the present invention can serve as a safety control tower, specifically, supports data exchange by forming a network between databases, and enables information on the world to be grasped at a glance in the case of global multinational clinical trials or many items of partner members. In this regard, the pharmacovigilance platform may further support functions such as setting a data exchange schedule and allocation of a task to a person in charge.

According to an embodiment, a plurality of servers may be operated as one server for a specific item based on database and network characteristics. That is, the servers of member companies that are partners with each other may be used as a single team. Specifically, functions configurable within one server may be implemented by aligning functions of a plurality of connected servers.

According to a specific embodiment, the CE step, QR step, MR step, and approval step may be shared between servers of different member companies. For example, server #2 of the partner company in country A collects cases in country A, performs intake & triage and CE (case entry) steps, and transmits it to server #1 of the partner company of country B which acts as a control tower. Thereafter, server #1 may transmit the result to server #2 after performing the QR step, MR step, and approval step. That is, server #1 may assign a regulatory authority reporting case to the reporting page of server #2.

According to a specific embodiment, division of labor between three or more servers may be achieved. For example, if the headquarter in country A, which is the control tower, is entrusted with the PV activities of branch offices in country B, when server #1 of the headquarter of the member company established in country A, server #2 of the branch offices established in country B, and server #3 of the partner company established in country B exist, server #3 collects cases, performs intake & triage and CE steps, and then transmits it to server #2. Thereafter, server #2 may perform the QR step and the MR step, and then transmit the result to server #1. When server #1 performs the approval step, generates the final report, and then transmits it to server #2, server #2 may transmit the final report to the server of the regulatory authority. A similar procedure may be performed with servers of branch offices established in other countries.

The procedure performed by aligning the functions of the plurality of servers as described above may be performed in an environment in which performance or similarity of characteristics of the artificial intelligence model between the plurality of servers is secured. To this end, a plurality of servers interworking with each other may be managed to have similar performance and compatible characteristics through learning data sharing, transfer learning, artificial neural network synchronization, and the like.

According to an embodiment, data exchange schedule setting, assignment of a task to a person in charge, regulatory authority reporting management, business continuity plan (BCP) management operated by national holidays/vacation/disaster situations, etc. may be performed in a timely manner. Therefore, the quality of pharmacovigilance can be improved. Here, for business continuity plan management, the platform determines when each user's task is expected to be stopped based on vacation plans of each user, holidays identified based on calendar information, and disaster information input by a manager, and determine and connect with other users (hereinafter referred to as 'backup users') who may back up the corresponding task. In order to select a backup user, the platform may generate a condition capable of backing up a user who will experience task interruption, search for a backup user meeting the generated condition, and send a request message to the searched backup user. In addition, the platform may provide information on the searched backup user to related users related to the user's task that will experience task interruption.

While the backup user is performing the backup task, the platform temporarily grants the backup user the necessary authority for backup. For temporary grant of authority, the platform establishes a secondary authority independent of the existing authority, and sets a period limit on the secondary authority. In this case, not only the authority of the user, but also the authority of access to data viewed, downloaded, and created during the backup task may have the same period limitation. That is, even if the data is generated by the backup user, the backup user cannot access the data after the authority period has elapsed.

According to the present invention, pharmacovigilance (PV) task can be evaluated and processed in medical aspects such as causality more consistently and accurately, and safety information and risks can be quickly detected and managed.

The effects obtainable in the present invention are not limited to the effects mentioned above, and other effects not mentioned herein can be clearly understood by those skilled in the art from the description.

The features briefly summarized above with respect to the present invention are only exemplary aspects of the detailed description of the present invention that follows, and do not limit the scope of the present invention.

Exemplary methods of the present invention are presented as a series of operations for clarity of explanation, but this is not intended to limit the order in which steps are performed, and the steps may be performed concurrently or in different orders, if necessary. In order to implement the method according to the present invention, other steps may be included in addition to the exemplified steps, other steps may be included except for some steps, or additional other steps may be included except for some steps.

Various embodiments of the present invention are not intended to list all possible combinations, but are intended to explain representative aspects of the present invention, and matters described in various embodiments may be applied independently or in combination of two or more.

In addition, various embodiments of the present invention may be implemented by hardware, firmware, software, or a combination thereof. In the case of implementing the present invention by hardware, the embodiments of the present invention may be achieved by one or more Application Specific Integrated Circuits (ASICs), Digital Signal Processors (DSPs), Digital Signal Processing Devices (DSPDs), Programmable Logic Devices (PLDs), Field Programmable Gate Arrays (FPGAs), general processors, controllers, microcontrollers, microprocessors, etc.

The scope of the present invention includes software or machine-executable instructions (e.g., operating systems, applications, firmware, programs, etc.) that cause operations according to methods of various embodiments to be executed on a device or computer, and a non-transitory computer-readable medium in which such software or instructions and the like are stored and executable on a device or computer.

## Claims

1. A method of operating a server for providing a pharmacovigilance (PV) platform and managing a first database (DB) of a first member company and a second DB of a second member company, the method comprising:
obtaining information on an agreement between the first member company and the second company for a product;
establishing a connection for data sharing between the first DB and the second DB;
setting a rule for data sharing; and
performing control to share data related to the product between the first DB and the second DB based on the rule.

2. The method of claim 1, wherein the performing control to share the data related to the product comprises sending a notification message indicating that adverse event data of the product has been generated.

3. The method of claim 1, wherein the performing control to share the data related to the product comprises sending report data for reporting an adverse event for the product.

4. The method of claim 1, wherein the rule defines a scope of data shared with the second server, a condition for sharing of the data and at least one data item according to the condition.

5. The method of claim 1, further comprising:
determining tasks for the adverse event upon a notification message indicating that the adverse event data of the product has been generated is received; and
setting a schedule and workflow of the tasks.

6. The method of claim 5, wherein the workflow designates a task for each DB or for each user.

7. The method of claim 5, wherein the tasks comprise a first task allocated to the first server and a second task allocated to the second server, and
wherein, when the second task is not completed within a time limit indicated by the schedule, a message for confirming progress of the second task is sent to the second DB.

8. The method of claim 7, further comprising performing the first task based on a result of the second task in response to receiving data notifying completion of the second task and including the result of the second task,
wherein the data notifying completion of the second task and including the result of the second task is generated in the second DB in response to confirmation of completion of the second task in the second DB based on the rule.

9. The method of claim 1, further comprising performing a reconciliation task of comparing at least one of a data reception history from the second DB or a data transmission history to the second DB with a record in the second DB, according to a predefined period.

10. A server for providing a pharmacovigilance (PV) platform and managing a first database (DB) of a first member company and a second DB of a second member company, the server comprising:
a storage configured to store information necessary for operation of the server;
a communication unit configured to transmit and receive data; and
a processor connected to the storage and the communication unit,
wherein the processor is configured to:
obtain information on an agreement between the first member company and the second company for a product;
establish a connection for data sharing between the first DB and the second DB;
set a rule for data sharing; and
perform control to share data related to the product between the first DB and the second DB based on the rule.
